# FASCICULE DE BREVET EUROPEEN

(11) **EP 2 855 660 B1**
(45) Date de publication et mention de la délivrance du brevet: **17.07.2019**
(21) Numéro de dépôt: 13728494.9
(22) Date de dépôt: 27.05.2013
(51) Int. Cl.: C12N 1/16, C12N 15/01

(54) **PROCEDE D'OBTENTION DE SOUCHES DE LEVURE AMELIOREES**
VERFAHREN ZUR GEWINNUNG VON VERBESSERTEN HEFESTÄMMEN
METHOD FOR OBTAINING IMPROVED STRAINS OF YEAST

(30) Priorité: 01.06.2012 FR 1255118
(43) Date de publication de la demande: 08.04.2015
(73) Titulaire: Lesaffre et Compagnie, 75001 Paris (FR)
(72) Inventeur: DESFOUGERES, Thomas, F-59960 Neuville En Ferrain (FR); PIGNEDE, Georges, F-59700 Marcq-En-Baroeul (FR)
(74) Mandataire: Cabinet Plasseraud
(86) Numéro de dépôt international: PCT/FR2013/051160
(87) Numéro de publication internationale: WO 2013/178918

(56) Documents cités:
- WO-A1-02/29009
- WO-A1-2009/112940
- WO-A1-2011/128552
- FR-A1- 2 679 249
- YAMANE SHOJI ET AL: "Region specificity of chromosome III on gene expression in the yeast Saccharomyces cerevisiae", JOURNAL OF GENERAL AND APPLIED MICROBIOLOGY, vol. 44, no. 4, août 1998 (1998-08), pages 275-281, XP002688129, ISSN: 0022-1260 cité dans la demande

## Description

### DOMAINE TECHNIQUE

La présente invention concerne une méthode d'obtention et de sélection de souches de levure améliorées.

### ARRIERE-PLAN TECHNOLOGIQUE

Grâce aux progrès de la biologie moléculaire, de la biologie cellulaire et de la génétique, de nombreux outils sont maintenant disponibles pour modifier une cellule, qu'il s'agisse d'une cellule procaryote ou d'une cellule eucaryote telle que la levure.

Il est, par exemple, possible d'obtenir une cellule qui produit ou utilise de nouveaux composés, ou encore d'améliorer les propriétés d'une cellule, en utilisant des techniques de modification de gènes, que ce soit par intégration, délétion ou disruption de séquences d'ADN, par mutagenèse dirigée ou aléatoire.

L'homme du métier dispose également de techniques plus anciennes telles que les techniques de croisement.

L'homme du métier peut également combiner ces techniques, comme par exemple réaliser une mutagenèse sur une souche de levure génétiquement modifiée, ou modifier génétiquement une souche de levure obtenue par croisement.

Cependant, l'homme du métier est parfois limité par la lourdeur, le temps nécessaire et le manque d'efficacité des techniques connues pour obtenir la cellule améliorée souhaitée.

La modification d'une souche de levure permet d'obtenir des levures avec des propriétés différentes ou améliorées, pouvant être utilisées dans de nombreuses applications possibles, parmi lesquelles la panification, l'alimentation, la santé, la production de composés, par exemple l'alcool, la production d'extraits de levure.

Le croisement de souches de levure est une technique largement utilisée, mais qui nécessite souvent des optimisations pour augmenter les chances d'obtenir la souche de levure souhaitée, comme par exemple la sélection des ségrégeants sur la base de propriétés particulières.

Toutefois, le manque d'efficacité du croisement de souches de levure est loin d'être résolu pour tous les cas de figure. Par exemple, il est difficilement envisageable d'utiliser la technique de croisement à partir d'une souche de levure qui a subi plusieurs modifications génétiques. En effet, la probabilité d'obtenir des ségrégeants ayant toutes les modifications génétiques de la souche de départ diminue avec le nombre de modifications génétiques.

Aussi, existe-t-il toujours un besoin de fournir de nouveaux procédés permettant d'obtenir des souches de levure améliorées, ces procédés étant plus rapides, simples de mise en oeuvre et permettant une sélection plus efficace des souches de levure possédant les améliorations souhaitées.

WO 2009/112910 décrit une cassette d'intégration de gènes dans le génome d'une souche de levure comprenant : au moins deux cassettes d'expression, chaque cassette d'expression comprenant un gène d'intérêt encadré par un promoteur et un terminateur, et deux séquences de recombinaison homologues d'une région liée à un locus d'expression du type sexuel et encadrant lesdites cassettes d'expression. Yamane Shoji et al. (J. Gen. Appl. Microbiol., 1998, 44(4): 275-281) décrit l'utilisation d'une région liée à un locus d'expression du type sexuel pour l'intégration d'un gène d'intérêt dans une souche de levure.

### RESUME DE L'INVENTION

La présente invention concerne un procédé d'obtention et de sélection d'une souche de levure améliorée, par intégration d'au moins deux gènes d'intérêt puis croisement, comprenant les étapes de:
- sélection d'une souche de levure X hétérothallique et hétérozygote au niveau du locus d'expression du type sexuel,
- intégration dans le génome de ladite souche de levure X d'au moins deux gènes d'intérêt dans une région liée à un locus d'expression du type sexuel, pour obtenir une souche de levure X génétiquement modifiée, et
- croisement de la souche de levure X génétiquement modifiée avec une souche de levure Y, pour obtenir un hybride,
où l'intégration est effectuée avec une cassette d'intégration de gènes, ou un vecteur comprenant une cassette d'intégration de gènes, la cassette d'intégration de gènes comprenant:
- au moins deux cassettes d'expression, chaque cassette d'expression comprenant un gène d'intérêt encadré par un promoteur et un terminateur, et
- deux séquences de recombinaison homologues d'une région liée à un locus d'expression du type sexuel et encadrant lesdites cassettes d'expression,
caractérisée en ce que ladite région liée à un locus d'expression du type sexuel est une région située à moins de 5 cM dudit locus d'expression du type sexuel,
et où l'étape de croisement de la souche de levure X génétiquement modifiée avec une souche de levure Y comprend les étapes de:
a) sporulation de la souche de levure X génétiquement modifiée et sélection, en une seule étape, d'au moins un ségrégeant du type sexuel correspondant à l'intégration des aux moins deux gènes d'intérêt, pour obtenir au moins un ségrégeant X,
b) sporulation de la souche de levure Y et sélection d'au moins un ségrégeant de la souche de levure Y de type sexuel compatible avec le ségrégeant X, pour obtenir un ségrégeant Y, et
c) hybridation d'au moins un ségrégeant X avec au moins un ségrégeant Y, pour obtenir au moins un hybride.

Dans certains modes de réalisation du procédé selon la présente invention, la région liée à un locus d'expression du type sexuel est une région située à moins de 10500 paires de base dudit locus d'expression du type sexuel.

Dans certains modes de réalisation, la cassette d'intégration utilisée dans le procédé selon l'invention comprend en outre, entre les deux séquences de recombinaison, deux séquences lox encadrant une cassette d'expression, ladite cassette d'expression comprenant un marqueur de sélection encadré par un promoteur et un terminateur.

Dans certains modes de réalisation, la région liée à un locus d'expression du type sexuel est comprise dans le gène BUD5, dans le gène TAF2 et/ou dans le locus d'expression du type sexuel.

Dans certains modes de réalisation, la souche de levure Y utilisée dans un procédé selon l'invention comprend au moins un gène d'intérêt dans une région liée à un locus d'expression du type sexuel.

Dans certains modes de réalisation, la souche de levure Y a au moins un caractère d'intérêt.

Dans certains modes de réalisation, l'étape b) de croisement du procédé selon l'invention comprend en outre la sélection d'un ségrégeant de la souche de levure Y qui a tout ou partie dudit caractère d'intérêt, pour obtenir un ségrégeant Y.

Dans certains modes de réalisation, le procédé de l'invention comprend en outre une étape ultérieure de sélection d'au moins un hybride qui a tout ou partie dudit caractère d'intérêt de la souche de levure Y.

Le présent document décrit aussi une cassette d'intégration de gènes comprenant :
- au moins une cassette d'expression comprenant un gène d'intérêt encadré par un promoteur et un terminateur,
- deux séquences de recombinaison homologues d'une région liée à un locus d'expression du type sexuel et encadrant lesdites cassettes d'expression.

Est également décrit un vecteur comprenant une cassette d'intégration de gènes telle que définie ci-dessus.

Est également décrite ici une souche de levure comprenant au moins deux gènes d'intérêt intégrés dans une région liée au locus d'expression du même type sexuel, de préférence transformée avec une cassette d'intégration de gènes telle que définie ci-dessus ou un vecteur tel que défini ci-dessus.

Le présent document décrit aussi une souche de levure améliorée susceptible d'être obtenue par le procédé d'obtention d'une souche de levure améliorée tel que défini ci-dessus ; une souche de levure dérivée d'une telle souche de levure améliorée, ladite souche de levure dérivée comprenant ledit ou lesdits gènes d'intérêt de la souche de levure X ; une levure obtenue par culture d'une telle souche de levure améliorée ou d'une telle souche de levure dérivée ; et l'utilisation d'une région liée au locus d'expression du type sexuel pour l'intégration d'au moins un gène d'intérêt dans une souche de levure destinée à être croisée avec une souche de levure.

### DESCRIPTION DES FIGURES

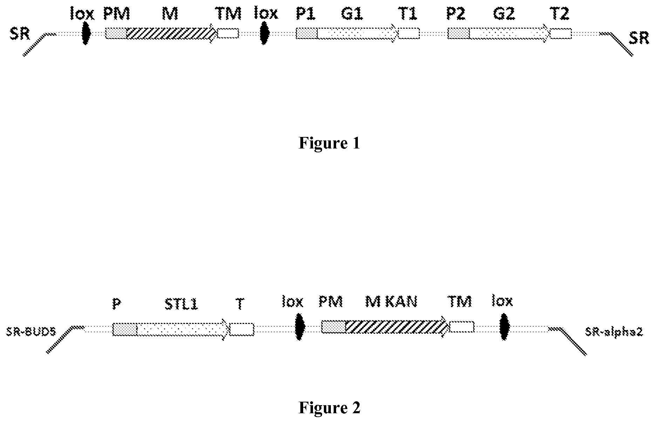

Figure 1 : Schéma d'une cassette d'intégration de gènes comprenant :
   - deux cassettes d'expression, chaque cassette d'expression comprenant un gène d'intérêt (respectivement G1 et G2) encadré par un promoteur P (respectivement P1 et P2) et un terminateur (respectivement T1 et T2),
   - deux séquences lox encadrant une cassette d'expression, ladite cassette d'expression comprenant un marqueur de sélection (M) encadré par un promoteur (PM) et un terminateur (TM),
   - deux séquences de recombinaison (SR) homologues d'une région liée à un locus d'expression du type sexuel et encadrant lesdites deux cassettes d'expression et lesdites deux séquences lox.
Figure 2 : Schéma de la cassette d'expression utilisée dans l'exemple 1 pour intégrer le gène STL1 dans le génome de la souche de levure. La cassette d'expression comprend :
   - une cassette d'expression, comprenant le gène d'intérêt STL1 encadré par le promoteur pADH1 (P) et le terminateur tCYCl (T),
   - deux séquences lox encadrant une cassette d'expression, ladite cassette d'expression comprenant un marqueur de sélection Kanamycine (M KAN) encadré par un promoteur (PM) et un terminateur (TM),
   - deux séquences de recombinaison (SR-BUD5 et SR-alpha2) homologues respectivement de l'extrémité 5' du gène BUD5 et de l'extrémité 3' du gène alpha2.

### DESCRIPTION DETAILLEE DE L'INVENTION

La présente invention a ainsi pour objet de fournir un nouveau procédé rapide, efficace et simple de mise en oeuvre, permettant d'obtenir des souches de levure améliorées.

De manière tout à fait originale, les Inventeurs ont mis en évidence qu'il est possible de sélectionner efficacement les ségrégeants d'une souche de levure génétiquement modifiée en intégrant la ou les modifications génétiques dans une région génétiquement liée à un locus responsable du type sexuel.

Ainsi, après sporulation de la souche de levure génétiquement modifiée, la ou les modifications génétiques ségrégent conjointement et se situent toutes dans un ségrégeant du type sexuel correspondant.

Le procédé original selon l'invention est d'autant plus intéressant qu'il est applicable à tout gène, pour tout type d'amélioration possible et à toute souche de levure qui a un cycle haplodiplophasique.

Un cycle haplodiplophasique est un cycle de reproduction qui alterne une phase haploïde et une phase diploïde et au cours duquel l'organisme considéré peut se multiplier par mitose aussi bien à l'état haploïde qu'à l'état diploïde.

Une souche de levure qui a un cycle haplodiplophasique est appelée souche de levure haplodiplophasique par la suite.

Le procédé selon l'invention n'est donc pas limité à un ou des gènes d'intérêt particuliers.

L'expression « souche de levure » désigne une population relativement homogène de cellules de levure.

Une souche de levure est obtenue à partir de l'isolement d'un clone, un clone étant une population de cellules obtenue à partir d'une seule cellule de levure.

Le procédé selon l'invention peut être utilisé pour améliorer toute souche de levure haplodiplophasique.

Une souche de levure haplodiplophasique préférée est un hémi-ascomycète ou une souche du genre *Schizosaccharomyces.*

Une souche de levure haplodiplophasique hémi-ascomycète pouvant être utilisée dans la mise en oeuvre du procédé de l'invention est par exemple une souche de levure du genre *Saccharomyces, Candida, Pichia* ou *Yarrowia.*

Une telle souche de levure du genre *Saccharomyces* est par exemple choisie parmi *Saccharomyces bailii, Saccharomyces boulardii, Saccharomyces carlsbergensis, Saccharomyces cerevisiae, Saccharomyces delbrueckii, Saccharomyces exiguus, Saccharomyces fermentati, Saccharomyces florentinus, Saccharomyces fragilis, Saccharomyces fructuum, Saccharomyces kefir, Saccharomyces kluyveri, Saccharomyces rosei, Saccharomyces steineri* et *Saccharomyces uvarum.*

Une souche de levure du genre *Candida* utilisée dans un procédé de l'invention est par exemple choisie parmi *Candida albicans* et *Candida glabrata.*

Une souche de levure du genre *Pichia* utilisée dans un procédé de l'invention est par exemple choisie parmi *Pichia anomola, Pichia heedii, Pichia guilliermondii, Pichia kluyveri, Pichia membranifaciens, Pichia norvegensis, Pichia ohmeri, Pichia pastoris, Pichia subpelliculosa* et *Pichia stipitis.*

Une souche de levure du genre *Yarrowia* utilisée dans un procédé de l'invention est par exemple *Yarrowia lipolytica.*

Une souche de levure utilisée dans un procédé de l'invention est par exemple une souche de levure phylogénétiquement proche de *Yarrowia lipolytica,* par exemple *Candida deformans* ou *Candida Gali.*

Une souche de levure du genre *Schizosaccharomyces* utilisée dans un procédé de l'invention est par exemple *Schizosaccharomyces pombe.*

Le locus d'expression du type sexuel est utilisé ici pour désigner tout locus exprimé responsable du type sexuel dans une souche de levure haploïde et qui n'est pas exprimé dans la souche de levure diploïde correspondante.

Un locus exprimé responsable du type sexuel correspond donc à une région du génome d'une souche de levure qui possède l'ensemble nécessaire pour exprimer un type sexuel dans ladite souche de levure à l'état haploïde et qui n'est pas exprimé dans la souche de levure diploïde correspondante.

L'utilisation du terme « locus d'expression » est importante, car il existe dans certaines souches de levure, un ou plusieurs locus identiques au locus d'expression du type sexuel, mais qui sont silencieux.

Un locus d'expression du type sexuel peut comprendre plusieurs gènes.

Un locus d'expression sexuel comprend généralement deux allèles définissant chacun un type sexuel, appelé également haplotype.

Les cellules d'un type sexuel donné ne peuvent féconder que les cellules de l'autre type sexuel. Les cellules d'un type sexuel donné sont qualifiées ici de type sexuel compatible avec les cellules de l'autre type sexuel.

Le terme « allèle » est donc utilisé de manière large, puisque un locus d'expression du type sexuel comprend généralement plusieurs gènes.

Le locus d'expression du type sexuel englobe ainsi ici un locus MAT (*Mating-type*) et un locus MTL (*Mating-type-like*).

Par exemple, dans une souche de levure *Saccharomyces,* le locus d'expression du type sexuel est un locus MAT qui comprend deux allèles : MATa et MATalpha.

La souche de levure *Saccharomyces* comprend également des copies silencieuses des allèles MATa et MATalpha qui sont appelées respectivement HMRa et HMRalpha.

Dans une souche de levure *Schizosaccharomyces*, le locus d'expression du type sexuel est un locus MAT qui comprend deux allèles : MAT1-P et MAT1-M.

La souche de levure *Schizosaccharomyces* comprend également des copies silencieuses des allèles MAT P et MAT M qui sont appelées respectivement mat2-P et mat-2-M.

Dans une souche de levure *Candida,* le locus d'expression du type sexuel est un locus MTL (*Mating-Type-Like*).

La souche de levure *Candida* a généralement un allèle MTLa et un allèle MTLalpha.

Préférablement, le locus d'expression du type sexuel est le locus MAT de *Saccharomyces.*

Le locus MAT de *Saccharomyces cerevisiae* est par exemple situé sur le chromosome III, sur le grand bras dudit chromosome, entre les positions 190 000 paires de base et 210 000 paires de base à partir du télomère du bras court.

L'allèle MATalpha de la souche de levure *Saccharomyces cerevisiae* comprend le gène alpha1 et le gène alpha2.

L'allèle MATa de la souche de levure *Saccharomyces cerevisiae* comprend le gène a1 et le gène a2.

La ou les modifications génétiques de la souche de levure améliorée décrite ici peuvent être obtenues au moyen d'une cassette d'intégration de gènes divulguée ici.

Une cassette d'intégration de gènes est ainsi décrite ici, comprenant :
- au moins une cassette d'expression, comprenant un gène d'intérêt encadré par un promoteur et un terminateur, et
- deux séquences de recombinaison homologues d'une région liée à un locus d'expression du type sexuel et encadrant ladite ou lesdites cassettes d'expression.

Le terme « gène » désigne ici une séquence d'ADN codante, appelée également « cadre ouvert de lecture ».

Comme indiqué ci-dessus, le gène d'intérêt peut être toute séquence d'ADN codante que l'on souhaite exprimer dans une souche de levure.

Un promoteur est une séquence d'ADN nécessaire à l'initiation et au contrôle de la transcription.

Le promoteur est, de préférence, un promoteur de la souche de levure dans laquelle la cassette d'intégration de gènes est destinée à être intégrée.

Par exemple, le promoteur est un promoteur de *Saccharomyces* choisi parmi pADH1, pTDH3, pPGK1, pADH2, pPDC2, pPMA1 et pGPD1.

Le terme « pX » désigne le promoteur du gène X.

Un terminateur est une séquence d'ADN nécessaire à la terminaison de la transcription.

Le terminateur est, de préférence, un terminateur de la souche de levure dans laquelle la cassette d'intégration de gènes est destinée à être intégrée.

Par exemple, le terminateur est un terminateur de *Saccharomyces* choisi parmi tCYC1, tTAL1 et tTKL1.

Le terme « tX » désigne le terminateur du gène X.

Le locus d'expression du type sexuel est tel que défini ci-dessus.

Le locus d'expression du type sexuel est par exemple un locus MAT ou un locus MTL.

Les deux séquences de recombinaison homologues d'une région liée à un locus d'expression du type sexuel sont telles qu'elles permettent l'intégration de ladite ou desdites cassettes d'expression dans une région liée au locus d'expression du type sexuel.

Chacune de ces séquences de recombinaison est donc homologue d'une région liée à un locus d'expression du type sexuel.

Les deux séquences de recombinaison peuvent être homologues de séquences adjacentes de la région liée à un locus d'expression du type sexuel ou non.

Si les deux séquences de recombinaison ne sont pas homologues de séquences adjacentes, la séquence d'ADN comprise entre les régions homologues aux séquences de recombinaison est excisée au moment de l'intégration de la cassette d'intégration de gènes dans la souche de levure.

Les deux séquences de recombinaison sont telles que la fonction du locus d'expression du type sexuel n'est pas altérée après intégration de la cassette d'intégration de gènes dans le génome de la souche de levure, afin que les ségrégeants produits par sporulation de ladite souche de levure soient capables de s'hybrider avec des ségrégeants de type sexuel compatible.

L'expression « région liée à » est équivalente ici à l'expression « région génétiquement liée à ».

Une région liée à un locus d'expression du type sexuel est une région telle que le risque d'une recombinaison par enjambement, entre ladite région et ledit locus d'expression du type sexuel, au cours de la méiose, est très faible.

Le terme « enjambement » est synonyme ici de « crossing-over ».

De ce fait, en intégrant la cassette d'intégration de gènes dans une région liée à un locus d'expression du type sexuel, la ou les cassettes d'expression de ladite cassette d'intégration de gènes et ledit locus d'expression du type sexuel ont de fortes chances de ségréger conjointement au cours de la méiose, c'est-à-dire de se retrouver dans le même ségrégeant.

Une séquence de recombinaison est constituée, de préférence, d'au moins 15 paires de base, plus préférentiellement au moins 20 paires de base, plus préférentiellement au moins 50 paires de base, plus préférentiellement encore au moins 100 paires de base, plus préférentiellement encore au moins 200 paires de base.

Une séquence de recombinaison est par exemple constituée de 200 à 400 paires de base.

Est également décrite ici une cassette d'intégration de gènes comprenant :
- au moins deux cassettes d'expression, chaque cassette comprenant un gène d'intérêt encadré par un promoteur et un terminateur, et
- deux séquences de recombinaison homologues d'une région liée à un locus d'expression du type sexuel et encadrant lesdites cassettes d'expression.

En particulier, la cassette d'intégration de gènes telle que définie ci-dessus est caractérisée en ce que ladite région liée à un locus d'expression du type sexuel est une région située à moins de 5 cM dudit locus d'expression du type sexuel.

Par l'expression « région située à moins de 5 cM du locus d'expression du type sexuel », on entend une région située à moins de 5 cM de part et d'autre du locus d'expression du type sexuel.

Le centimorgan, noté cM, est l'unité de mesure de la distance entre deux gènes génétiquement liés.

Le centimorgan est défini comme la fréquence de recombinaison, lors de la méiose, entre deux gènes dont les locus sont sur un même chromosome et dont les allèles présents sur chaque chromosome définissent pour chacun d'eux, un haplotype.

Une région située à moins de 5 cM du locus d'expression du type sexuel signifie donc que la fréquence de recombinaison entre ladite région et ledit locus d'expression du type sexuel est de 5 %, c'est-à-dire 5 enjambements en moyenne sur cette distance pour 100 méioses.

La cassette d'intégration de gènes telle que définie ci-dessus peut également être caractérisée en ce que ladite région liée à un locus d'expression du type sexuel est une région située à moins de 4 cM, de préférence moins de 3 cM, plus préférentiellement moins de 2 cM, plus préférentiellement encore moins de 1 cM dudit locus d'expression du type sexuel.

La cassette d'intégration de gènes telle que définie ci-dessus peut également être caractérisée en ce que ladite région liée à un locus d'expression du type sexuel est une région située à moins de 10500 paires de base dudit locus d'expression du type sexuel.

Par l'expression « région située à moins de 10500 paires de base du locus d'expression du type sexuel », on entend une région située à moins de 10500 paires de base de part et d'autre du locus d'expression du type sexuel.

Par exemple, la cassette d'intégration de gènes telle que définie ci-dessus est caractérisée en ce que ladite région liée à un locus d'expression du type sexuel est une région située à moins de 10500 paires de base du locus MAT de *Saccharomyces cerevisiae.*

La cassette d'intégration de gènes telle que définie ci-dessus peut également être caractérisée en ce que ladite région liée à un locus d'expression du type sexuel est une région située à moins de 8500 paires de base, de préférence moins de 6500 paires de base, plus préférentiellement moins de 4500 paires de base, plus préférentiellement encore moins de 2500 paires de base dudit locus d'expression du type sexuel.

La cassette d'intégration de gènes comprend de préférence au moins un marqueur de sélection, afin de permettre la sélection des cellules de levure qui ont intégré ladite cassette d'intégration.

Préférablement, les souches de levure améliorées décrites ici ne comprennent pas de marqueur de sélection.

Le système cre-lox peut alors être utilisé afin d'éliminer facilement le ou les marqueurs de sélection utilisés dans la cassette d'intégration de gènes, tout en ne laissant dans le génome de la souche de levure qu'une cicatrice comprenant une séquence lox.

La cassette d'intégration de gènes telle que définie ci-dessus peut donc comprendre, entre lesdites deux séquences de recombinaison, deux séquences lox encadrant une cassette d'expression, ladite cassette d'expression comprenant un marqueur de sélection encadré par un promoteur et terminateur.

Ainsi, la cassette d'intégration de gènes décrite ici peut donc comprendre par exemple :
- au moins deux cassettes d'expression, chaque cassette d'expression comprenant un gène d'intérêt encadré par un promoteur et un terminateur,
- deux séquences lox encadrant une cassette d'expression, ladite cassette d'expression comprenant un marqueur de sélection encadré par un promoteur et terminateur, et
- deux séquences de recombinaison homologues d'une région liée à un locus d'expression du type sexuel et encadrant lesdites cassettes d'expression et lesdites deux séquences lox.

Les cassettes d'expression comprenant les gènes d'intérêt et la cassette d'expression comprenant le marqueur de sélection qui est encadrée par les séquences lox sont placées dans n'importe quel ordre souhaité à l'intérieur des séquences de recombinaison.

Un exemple d'une telle cassette d'intégration de gènes est donné à la figure 1.

Le marqueur de sélection est de préférence un marqueur de sélection dominant.

Un marqueur de sélection dominant est un gène dont une seule copie fonctionnelle suffit à la cellule pour lui permettre de se multiplier sur un milieu sélectif.

Le marqueur de sélection dominant est par exemple un gène de résistance à un antibiotique, un gène codant une enzyme exogène ayant une activité d'hydrolyse d'un polyholoside, ou un gène codant pour une acétamidase.

Le gène codant une enzyme exogène ayant une activité d'hydrolyse d'un polyholoside est par exemple un gène codant une enzyme ayant une activité mélibiase, une activité alpha-amylase ou une activité isomaltase.

Le gène de résistance à un antibiotique est par exemple choisi parmi le gène de résistance à la kanamycine, blasticidine, phléomycine, hygromycine ou nourséothricine.

Les deux séquences lox permettent d'exciser le marqueur de sélection par utilisation d'une recombinase Cre.

Les deux séquences lox ont par exemple la séquence loxP de 34 paires de base suivante: ATAACTTCGTATAATGTATGCTATACGAAGTTAT (SEQ ID N0:1).

Dans certains cas, une cassette d'intégration de gènes telle que définie ci-dessus comprend au moins trois cassettes d'expression, chaque cassette d'expression comprenant un gène d'intérêt encadré par un promoteur et un terminateur.

La cassette d'intégration de gènes peut par exemple comprendre trois cassettes d'expression, quatre cassettes d'expression, cinq cassettes d'expression, ou plus de cinq cassettes d'expression, chaque cassette d'expression comprenant un gène d'intérêt encadré par un promoteur et un terminateur.

La cassette d'intégration de gènes telle que définie ci-dessus peut avantageusement être caractérisée en ce que ladite région liée à un locus d'expression du type sexuel comprend le gène BUD5, le gène TAF2 et/ou le locus d'expression du type sexuel.

En particulier, la cassette d'intégration de gènes telle que définie ci-dessus peut avantageusement être caractérisée en ce que ladite région liée à un locus d'expression du type sexuel est comprise dans le gène BUD5, dans le gène TAF2 et/ou dans le locus d'expression du type sexuel.

La cassette d'intégration de gènes telle que définie ci-dessus peut être caractérisée en ce que les séquences de recombinaison sont homologues d'une région comprise dans le gène BUD5, dans le gène TAF2 et/ou dans le locus d'expression du type sexuel.

Le gène BUD5 est également référencé sous le numéro YCR038C.

Le gène BUD5 a déjà été utilisé par le Demandeur comme site d'intégration de gènes, car il s'agit d'une région qui s'est révélée très favorable à l'expression d'un gène rapporteur en présentant un fort niveau d'activité bêta-glucosidase dans les cellules de levure ainsi transformées (*cf.* Yamane et al., J. Gen. Appl. Microbiol, 1998, 44, 275-281). Ainsi, la région du génome correspondant au gène BUD5 a été utilisée uniquement dans le but d'obtenir une forte expression du gène intégré.

Le gène TAF2 est également référencé sous le numéro YCR042C.

Une des séquences de recombinaison peut par exemple être homologue de l'extrémité 3' ou de l'extrémité 5' du locus d'expression sexuel.

Une séquence de recombinaison est par exemple homologue d'une région comprise dans le gène BUD5 ou dans le gène TAF2, et l'autre séquence de recombinaison est par exemple homologue d'une région comprise dans le locus d'expression du type sexuel.

Dans un autre exemple, les deux séquences de recombinaison sont homologues d'une région comprise dans le gène BUD5 ou d'une région comprise dans le gène TAF2.

Une séquence de recombinaison peut également être homologue d'une région chevauchant le gène BUD 5 et le locus d'expression du type sexuel, ou encore d'une région chevauchant le gène TAF2 et le locus d'expression du type sexuel.

Préférablement, une séquence de recombinaison est homologue du gène BUD5 et l'autre séquence de recombinaison est homologue du locus MAT.

Par exemple, une séquence de recombinaison est homologue du gène BUD5 de *Saccharomyces,* de préférence de la région 5' du gène BUD5, et l'autre séquence de recombinaison est homologue du gène alpha2 de *Saccharomyces,* de préférence de la région 3' du gène alpha2.

Est également décrit ici un vecteur comprenant une cassette d'intégration de gènes telle que définie ci-dessus.

Le terme « vecteur » désigne ici une molécule d'ADN capable d'autoréplication chez *E. Coli.*

Un vecteur est par exemple choisi parmi un plasmide, un cosmide, un chromosome artificiel de bactérie appelé également BAC, un chromosome artificiel dérivé du bactériophage Pl appelé également PAC, un vecteur dérivé de virus.

Est également décrite ici une souche de levure comprenant au moins deux gènes d'intérêt intégrés dans une région liée au locus d'expression du même type sexuel.

Une telle souche de levure constitue un produit intermédiaire de la mise en oeuvre du procédé d'obtention de souches de levure améliorées selon l'invention.

La souche de levure comprenant au moins deux gènes d'intérêt intégrés dans une région liée au locus d'expression du même type sexuel est hétérozygote au niveau du locus d'expression du type sexuel, c'est-à-dire qu'elle possède au moins un allèle de chacun des types sexuels.

La souche de levure comprenant au moins deux gènes d'intérêt intégrés dans une région liée au locus d'expression du même type sexuel est une souche de levure haplodiplophasique.

Une souche de levure haplodiplophasique pouvant être utilisée dans la mise en oeuvre d'un procédé l'invention est telle que définie ci-dessus.

Une souche de levure haplodiplophasique préférée est une souche de *Saccharomyces,* de préférence *Saccharomyces cerevisiae.*

Le locus d'expression du type sexuel est tel que défini ci-dessus.

Les gènes d'intérêt sont intégrés dans une région liée au locus d'expression du même type sexuel, c'est-à-dire du même allèle.

Dans le cas d'une souche de *Saccharomyces,* les gènes d'intérêt sont intégrés dans une région liée au locus MAT du même type sexuel, c'est-à-dire au locus MAT du type MATa ou au locus MAT du type MATalpha.

Est également décrite ici une souche de levure comprenant au moins trois gènes d'intérêt, par exemple trois gènes d'intérêt, quatre gènes d'intérêt, cinq gènes d'intérêt, ou plus de cinq gènes d'intérêt.

Est décrite en particulier ici une souche de levure telle que définie ci-dessus comprenant au moins deux gènes d'intérêt intégrés dans une région liée à un locus d'expression du même type sexuel, transformée avec une cassette d'intégration de gènes telle que définie ci-dessus ou avec un vecteur tel que défini ci-dessus.

Est aussi décrit ici un procédé d'obtention d'une souche de levure améliorée, par intégration d'au moins un gène dans une souche de levure, puis croisement de ladite souche de levure ainsi obtenue avec une autre souche de levure.

Comme indiqué ci-dessus, le procédé est rapide, efficace et simple de mise en oeuvre. En particulier, le procédé permet d'améliorer l'efficacité et donc de diminuer la durée de l'étape de sélection des ségrégeants issus de chaque souche de levure utilisée pour le croisement, et ce, même en intégrant un seul gène.

Le procédé d'obtention d'une souche de levure améliorée, par intégration d'au moins un gène d'intérêt puis croisement, comprend les étapes de :
- sélection d'une souche de levure X hétérothallique et hétérozygote au niveau du locus d'expression du type sexuel,
- intégration dans le génome de ladite souche de levure X d'au moins un gène d'intérêt dans une région liée à un locus d'expression du type sexuel, pour obtenir une souche de levure X génétiquement modifiée, et
- croisement de la souche de levure X génétiquement modifiée avec une souche de levure Y, pour obtenir un hybride.

Le procédé est applicable à toute souche de levure X haplodiplophasique.

Une souche de levure haplodiplophasique pouvant être utilisée dans la mise en oeuvre du procédé est telle que définie ci-dessus.

Une souche de levure haplodiplophasique préférée est une souche de *Saccharomyces,* de préférence *Saccharomyces cerevisiae.*

Le locus d'expression du type sexuel est tel que défini ci-dessus.

La souche de levure X est hétérozygote au niveau du locus d'expression du type sexuel, c'est-à-dire qu'elle possède au moins un allèle de chacun des types sexuels.

Par exemple, dans le cas d'une souche de *Saccharomyces,* la souche de levure X possède au moins un allèle MATalpha et au moins un allèle MATa.

Une souche de levure hétérothallique est une souche de levure dont le type sexuel est intrinsèquement stable.

En particulier, une souche de *Saccharomyces* hétérothallique est une souche de levure qui ne possède pas d'activité endonucléase.

Une souche de levure qui ne possède pas d'activité endonucléase Ho est qualifiée de génotype Ho-.

L'activité d'une endonucléase Ho peut en effet permettre le changement du type sexuel en changeant la composition génétique du locus du type sexuel.

L'étape d'intégration est de préférence effectuée en utilisant une cassette d'intégration de gènes telle que définie ci-dessus, par exemple au moyen d'un vecteur tel que défini ci-dessus.

L'étape d'intégration peut comprendre l'intégration dans le génome de la souche de levure X d'au moins deux gènes d'intérêt dans une région liée au locus d'expression du même type sexuel, pour obtenir une souche de levure X génétiquement modifiée.

L'étape d'intégration peut également comprendre l'intégration dans le génome de la souche de levure X d'au moins trois gènes d'intérêt dans une région liée au locus d'expression du même type sexuel, par exemple trois gènes d'intérêt, quatre gènes d'intérêt, cinq gènes d'intérêt, ou plus de cinq gènes d'intérêt, pour obtenir une souche de levure X génétiquement modifiée.

Dans le cas d'une souche de *Saccharomyces,* les gènes d'intérêt sont intégrés dans une région liée au locus MAT du même type sexuel, c'est-à-dire soit du type MATa, soit du type MATalpha.

Lorsqu'au moins deux gènes d'intérêt sont intégrés, ils peuvent être intégrés en utilisant plusieurs cassettes d'intégration de gènes, par exemple une cassette d'intégration pour chaque gène ou pour plusieurs gènes, ou en utilisant une cassette d'intégration de gènes comprenant tous les gènes d'intérêt.

Ainsi, tous les gènes d'intérêt sont intégrés dans une région liée au locus d'expression du même type sexuel, mais ils ne sont pas nécessairement intégrés au même endroit dans le génome de la souche de levure.

La souche de levure X génétiquement modifiée est croisée avec une souche de levure Y, pour obtenir un hybride.

L'hybride correspond à la souche de levure améliorée décrite ci-dessus.

La souche de levure Y est également une souche de levure haplodiplophasique.

La souche de levure Y est de préférence du même genre, de préférence de la même espèce que la souche de levure X.

En particulier, la souche de levure X modifiée génétiquement peut être croisée avec la souche de levure X non modifiée. Dans ce cas, la souche de levure X non modifiée et la souche de levure Y sont identiques.

Ainsi, les ségrégeants issus de la sporulation de la souche de levure X peuvent être sélectionnés facilement, en une seule étape, par rapport au type sexuel correspondant à l'intégration des gènes.

En effet, il n'y a pas besoin d'évaluer à la fois le type sexuel du ségrégeant de la souche de levure X, en plus de la présence du ou des gènes d'intérêt.

La détermination du type sexuel d'un ségrégeant est effectuée par les techniques bien connues de l'homme du métier, par exemple par PCR ou encore par croisement avec une souche de levure haploïde de type sexuel connu (l'obtention d'un diploïde indique que le ségrégeant de départ est du type sexuel compatible).

Ensuite, l'étape de sélection des ségrégeants de la souche de levure Y est hautement simplifiée, puisque seuls les ségrégeants du type sexuel compatible avec le ségrégeant X sont analysés.

Le procédé décrit ici présente donc des avantages dès l'intégration d'un gène d'intérêt.

A partir de deux gènes d'intérêt, le procédé décrit ici est encore plus avantageux, car en plus de ce qui est indiqué précédemment :
- les gènes d'intérêt de la souche de levure X ségrégent tous dans le ségrégeant du type sexuel correspondant à l'intégration desdits gènes, au lieu de se répartir de manière aléatoire entre chaque ségrégeant, ce qui augmente significativement les chances d'obtenir des ségrégeants qui comprennent tous les gènes d'intérêt, et
- la sélection des ségrégeants de la souche de levure X est grandement simplifiée, puisqu'il est possible de sélectionner, en une seule étape, les ségrégeants du type sexuel correspondant à l'intégration de tous les gènes d'intérêt.

En particulier, le procédé tel que défini ci-dessus peut être caractérisé en ce que la souche de levure X génétiquement modifiée est une souche de levure telle que définie ci-dessus comprenant au moins deux gènes d'intérêt intégrés dans une région liée à un locus d'expression du même type sexuel, de préférence transformée avec une cassette d'intégration telle que définie ci-dessus ou avec un vecteur tel que défini ci-dessus.

L'étape de croisement est réalisée selon les techniques classiques, comme celles enseignées dans le chapitre 7 « Sporulation and Hydridization of Yeast » par R.R. Fowell, de l'ouvrage de référence « The Yeasts », Volume 1, édité par A.H. Rose et J. S. Harrison, 1969-Academic Press.

Ainsi, un procédé tel que défini ci-dessus peut être caractérisé en ce que l'étape de croisement de la souche de levure X génétiquement modifiée avec une souche de levure Y comprend les étapes de:
a) sporulation de la souche de levure X génétiquement modifiée et sélection d'au moins un ségrégeant du type sexuel correspondant à l'intégration du ou desdits gènes d'intérêt, pour obtenir au moins un ségrégeant X,
b) sporulation de la souche de levure Y et sélection d'au moins un ségrégeant de la souche de levure Y de type sexuel compatible avec le ségrégeant X, pour obtenir un ségrégeant Y, et
c) hybridation d'au moins un ségrégeant X avec au moins un ségrégeant Y, pour obtenir au moins un hybride.

Dans certains cas, un procédé tel que défini ci-dessus peut être caractérisé en ce que la souche de levure Y comprend au moins un gène d'intérêt intégré dans une région liée à un locus d'expression du type sexuel.

La souche de levure Y peut comprendre au moins deux gènes d'intérêt, par exemple deux gènes d'intérêt, trois gènes d'intérêt, quatre gènes d'intérêt, cinq gènes d'intérêt, ou plus de cinq gènes d'intérêt.

Avantageusement, le ou les gènes d'intérêt de la souche de levure Y peuvent être intégrés dans une région liée à un locus d'expression du type sexuel compatible avec le type sexuel du ségrégant X.

Alternativement, le ou les gènes d'intérêt de la souche de levure Y peuvent être avantageusement intégrés dans une région liée à un locus d'expression du même type sexuel que celui du ségrégant X.

Dans un tel cas, afin d'obtenir un ségrégeant Y comprenant le ou lesdits gènes d'intérêt de la souche de levure Y qui soit de type sexuel compatible avec un ségrégeant X comprenant le ou lesdits gènes d'intérêt de la souche de levure X, il est possible de changer le type sexuel du locus d'expression sexuel correspondant à l'intégration du ou desdits gènes d'intérêt de la souche de levure X ou de la souche de levure Y, selon les techniques bien connues de l'homme du métier, par exemple par utilisation d'un plasmide contenant le gène HO qui va exprimer l'endonucléase HO dans la souche de levure (cf. Herskowitz, I. et Jensen R. E., 1991, Methods Enzymol 194, 132-46).

Les ségrégeants issus de la sporulation de la souche de levure Y comprenant au moins un gène d'intérêt peuvent être sélectionnés facilement, en une seule étape, par rapport au type sexuel correspondant à l'intégration du ou des gènes d'intérêt.

Le procédé décrit ici peut comprendre le croisement d'une souche de levure X comprenant un ou des gènes d'intérêt avec une souche de levure Y qui présente au moins un caractère d'intérêt.

Ainsi, dans certains cas, le procédé tel que défini ci-dessus, est caractérisé en ce que la souche de levure Y a au moins un caractère d'intérêt.

Un caractère d'intérêt au sens utilisé ici est génétiquement transmissible.

Le caractère d'intérêt peut être dominant ou co-dominant.

Un caractère d'intérêt dominant est un caractère transmissible en une seule génération.

Un caractère d'intérêt co-dominant est un caractère transmissible partiellement en une seule génération.

Des exemples de caractère d'intérêt sont :
- la résistance et/ou la tolérance à un stress et/ou à un inhibiteur, tels que la pression osmotique, le pH, la présence d'acides organiques, une concentration élevée en alcool, la présence de composés aromatiques tels que les terpènes, un antibiotique, une température élevée, la congélation, le séchage et leur combinaison, et/ou
- un rendement élevé en éthanol en fermentation et/ou une force fermentative élevée en panification et/ou un rendement élevé de production de biomasse.

Les exemples de caractères d'intérêt indiqués ci-dessus ne sont pas limitatifs.

Un procédé tel que défini ci-dessus peut être caractérisé en ce que l'étape b) de croisement comprend en outre la sélection d'un ségrégeant de la souche de levure Y qui a tout ou partie dudit caractère d'intérêt, pour obtenir un ségrégeant Y.

Un procédé tel que défini ci-dessus peut être caractérisé en ce qu'il comprend une étape ultérieure de sélection d'au moins un hybride qui a tout ou partie du caractère d'intérêt de la souche de levure Y.

La souche de levure X peut également avoir au moins un caractère d'intérêt.

Dans ce cas, parmi les ségrégeants de la souche de levure X du type sexuel correspondant à l'intégration du ou des gènes d'intérêt, sont sélectionnés les ségrégeants qui ont tout ou partie du caractère d'intérêt de la souche de levure X.

Un procédé tel que défini ci-dessus peut être caractérisé en ce qu'il comprend une étape ultérieure de sélection d'au moins un hybride qui a tout ou partie du caractère d'intérêt de la souche de levure X.

Est aussi décrite ici une souche de levure améliorée susceptible d'être obtenue par le procédé tel que défini ci-dessus.

Est également décrite ici une souche de levure dérivée d'une souche de levure améliorée telle que définie ci-dessus, ladite souche de levure dérivée comprenant ledit ou lesdits gènes d'intérêt de la souche de levure X.

Par l'expression « souche dérivée », on désigne une souche dérivée par un ou plusieurs croisements et/ou par mutation et/ou par transformation génétique.

Une souche dérivée par croisement peut être obtenue par croisement d'une souche tel que décrit plus haut avec la même souche, ou une autre souche selon l'invention, ou une autre souche quelconque.

Une souche dérivée par mutation peut être une souche ayant subi au moins une mutation spontanée dans son génome ou au moins une mutation induite par mutagenèse. La ou les mutations d'une souche dérivée peuvent être silencieuses ou non.

Par l'expression « mutagenèse », on désigne à la fois la mutagenèse aléatoire obtenue par l'application d'un rayonnement (par exemple UV), ou par des agents chimiques mutagènes, et la mutagenèse insertionnelle ou dirigée, par transposition ou par intégration d'un fragment d'ADN exogène.

Une souche dérivée par transformation génétique est une souche dans laquelle a été introduite une séquence d'ADN qui est, de préférence, apportée par un plasmide ou intégrée directement dans le génome.

Est également décrite ici une levure obtenue par culture d'une souche de levure améliorée telle que définie ci-dessus ou d'une souche de levure dérivée telle que définie ci-dessus.

Les levures sont produites à partir de souches de levure large spectre, notamment comme décrit dans le livre de référence « Yeast Technology », 2ème édition, 1991, G. Reed et T.W. Nagodawithana, publié par Van Nostrand Reinhold, ISBN 0-442-31892-8.

La multiplication des levures, à l'échelle industrielle, comprend en général au moins les deux premières étapes de l'ensemble des étapes suivantes :
- multiplication d'une souche de levure en plusieurs stades, d'abord en semi-anaérobiose, puis en aérobiose,
- séparation par centrifugation de la levure ainsi produite de son milieu de culture, pour obtenir une crème de levure liquide contenant environ entre 12 et 25% de matière sèche, voire une quantité plus élevée de matière sèche si la crème de levure est mélangée avec des produits osmolytes,
- filtration de la crème de levure liquide ainsi obtenue, en général sur un filtre rotatif sous vide, pour obtenir une levure fraîche déshydratée contenant de 26% à 35% de matière sèche,
- malaxage de ladite levure fraîche déshydratée, pour obtenir une masse homogène,
- extrusion de la levure ainsi obtenue, pour obtenir :
- une levure pressée sous forme de pains de levure fraîche ou de levure fraîche émiettée, contenant environ 30% de matière sèche, ou
- une levure sous forme de particules, en général de granules, si la levure est destinée à être séchée,
- éventuellement, séchage de manière ménagée, dans un courant d'air chaud, par exemple par fluidisation, des particules de levures obtenues par extrusion pour obtenir de la levure sèche.

Est aussi décrite ici l'utilisation d'une région liée à un locus d'expression du type sexuel pour l'intégration d'au moins un gène d'intérêt dans une souche de levure destinée à être croisée avec une souche de levure.

Le locus d'expression du type sexuel est tel que défini ci-dessus.

L'utilisation telle que définie ci-dessus peut être caractérisée en ce que la souche de levure dans laquelle est intégrée au moins un gène d'intérêt est une souche de levure haplodiplophasique, par exemple telle que définie ci-dessus.

Une souche de levure haplodiplophasique préférée est une souche de *Saccharomyces,* de préférence *Saccharomyces cerevisiae.*

Dans certains cas, l'utilisation telle que définie ci-dessus est caractérisée en ce que la souche de levure dans laquelle au moins un gène d'intérêt est intégré, est destinée à être croisée avec une autre souche de levure, ou la même souche de levure avant l'intégration du ou des gènes d'intérêt.

Dans d'autres cas, l'utilisation telle que définie ci-dessus est caractérisée en ce que la souche de levure dans laquelle au moins un gène d'intérêt est intégré est destinée à être croisée avec une souche de levure qui comprend également au moins un gène d'intérêt intégré dans une région liée au locus d'expression du type sexuel.

Dans encore d'autres cas, l'utilisation telle que définie ci-dessus est caractérisée en ce que la souche de levure dans laquelle au moins un gène d'intérêt est intégré est hétérozygote au niveau du locus d'expression du type sexuel, c'est-à-dire qu'elle possède au moins un allèle de chacun des types sexuels.

Est aussi décrite ici l'utilisation telle que définie ci-dessus d'une région liée à un locus d'expression du type sexuel pour l'intégration d'au moins deux gènes d'intérêt dans une souche de levure destinée à être croisée avec une souche de levure, de préférence au moins trois gènes d'intérêt, par exemple trois gènes d'intérêt, quatre gènes d'intérêt, cinq gènes d'intérêt, ou plus de cinq gènes d'intérêt.

Est également décrite ici l'utilisation telle que définie ci-dessus dans laquelle la région liée à un locus d'expression du type sexuel est une région située à moins de 5 cM, de préférence moins de 4 cM, de préférence moins de 3 cM dudit locus d'expression du type sexuel, plus préférentiellement moins de 2 cM, plus préférentiellement encore moins de 1 cM.

L'utilisation telle que définie ci-dessus peut aussi être caractérisée en ce que la région liée à un locus d'expression du type sexuel est une région située à moins de 10500 paires de base, de préférence moins de 8500 paires de base, de préférence moins de 6500 paires de base, plus préférentiellement moins de 4500 paires de base, plus préférentiellement encore moins de 2500 paires de base dudit locus d'expression du type sexuel.

D'autres caractéristiques et avantages de l'invention apparaîtront encore mieux à la lecture de l'exemple de réalisation suivant qui illustre l'invention sans la limiter.

### EXEMPLE : OBTENTION D'UNE SOUCHE LEVURE AMELIOREE PAR CROISEMENT D'UNE SOUCHE DE LEVURE GENETIQUEMENT MODIFIEE AVEC UNE AUTRE SOUCHE DE LEVURE

### Matériel et Méthodes

### (i) Production de levure

La production de levure est menée de la manière décrite dans l'ouvrage « Yeast technology », 2ème édition, 1991, G. Reed et T.W. Nagodawithana, publié par Van Nostrand Reinhold, ISBN 0-442-31892-8, en particulier en réalisant deux pré-cultures successives suivies d'une culture en fed-batch. La levure ainsi obtenue est ensuite séchée pour obtenir une levure sèche.

### (ii) Mesure de la force fermentative

La force fermentative d'une levure correspond au volume de CO₂ (en ml) produit par la levure en fermentation dans une pâte.

La force fermentative des levures est mesurée sur levure sèche, dans une pâte sans sucre contenant 20g de farine.

La levure sèche, dans une quantité égale à 160 mg de matières sèches, est réhydratée dans 6 ml d'eau distillée à 38°C pendant 15 minutes. On ajoute ensuite 9 ml d'un mélange d'eau et 405 mg de NaCl. On obtient ainsi une suspension de levures dans une solution aqueuse contenant 27 g/l de NaCl.

La farine et ladite suspension de levures sont malaxées pendant 40 secondes dans un pétrin, de manière à obtenir une pâte qui est ensuite placée dans un récipient mis au bain-marie à 30°C 13 minutes après le début du malaxage, le récipient contenant la pâte est fermé hermétiquement.

Le volume de dégagement gazeux est mesuré au cours de la 1ère heure et au cours de la 2ème heure, au moyen d'un Risograph (National Manufacturing, Lincoln, Nebraska). On indique ensuite le volume total en ml sur 2 heures à 30°C.

### (iii) Préparation de la souche de levure X génétiquement modifiée

La souche de levure X est une souche de *Saccharomyces cerevisiae* modifiée génétiquement pour pouvoir se multiplier, en condition aérobie, dans un milieu de culture contenant un mélange de glycérol et de sucre, en consommant le glycérol.

A cet effet, le gène STL1 est intégré dans une région comprise entre l'extrémité 5' du gène BUD5 et l'extrémité 3' du gène alpha2 (situé dans le locus MATalpha) en utilisant la cassette d'intégration de gènes représentée dans la figure 2.

Le gène STL1 est sous contrôle du promoteur pADH1 et le terminateur est tCYC1.

La cassette d'intégration comprend le marqueur de sélection kanamycine, conférant une résistance à la généticine, encadré d'un promoteur et d'un terminateur et de deux séquences loxP.

La première séquence de recombinaison est homologue d'une région comprise dans les 600 premières paires de base du gène BUD5 et la deuxième séquence de recombinaison est homologue d'une région comprise de la paire de base 150 à la fin du gène alpha2.

Le marqueur de sélection est utilisé pour sélectionner les souches de levure qui ont intégré le gène STL1.

L'intégration du gène STL1 au niveau du gène BUD5 et du gène aplha2 est vérifiée.

Le marqueur de sélection est ensuite excisé en utilisant la recombinase Cre.

La souche de levure ainsi obtenue correspond à la souche de levure F.

### (iv) Croisement

La souche de levure de départ F est croisée avec la souche de levure de départ G.

La souche de levure G est une souche de *Saccharomyces cerevisiae* performante en panification sur pâte sans sucre.

En particulier, la souche de levure G donne des levures qui ont une force fermentative supérieure ou égale à 150 ml en pâte sans sucre.

### Etape 1 : Croissance de la souche de levure

Une öse de la souche de départ (conservée à -80°C) est ensemencée en surface de la gélose d'une boîte de Pétri de milieu A.

La composition du milieu A est la suivante : 10 g d'extrait de levure, 10 g de peptone, 20 g de glucose, 20 g d'agar, pH 6,2 +/- 0,2, eau qsp IL.

La boîte de Pétri est ensuite mise en incubation pendant 24 heures à 30°C.

### Etape 2 : Sporulation sur du milieu 2

Une öse de la culture précédente est ensemencée en surface de la gélose d'une boîte de Pétri de milieu B.

La composition du milieu B est la suivante : 6,5g d'acétate de sodium, 15g d'agar, pH 6,5-7, eau qsp IL.

La boîte de Pétri est mise en incubation pendant 96 heures à 25°C. La biomasse obtenue en surface de la boîte est ensuite récoltée dans 500µL d'eau stérile. A 100µL de cette suspension sont ajoutés 25µl de zymolyase. La suspension est mise en incubation pendant 30 minutes à 30°C, avant d'être étalée sur boîte gélosée de milieu 1. De préférence, la dissection des tétrades est effectuée 20 minutes plus tard.

### Etape 3 : Dissection des tétrades

Les tétrades sont disséquées au micro-manipulateur SINGER, puis la boîte de pétri est mise en incubation pendant 48 heures à 30°C.

### Etape 4 : Conservation des ségrégeants

Les ségrégeants sont ensuite conservés à -80°C dans du milieu 1 contenant 20% de glycérol.

### Etape 5 : Sélection des ségrégeants de la souche de levure F

Les ségrégeants de la souche de levure F sont sélectionnés sur la base de la présence de l'allèle MATalpha qui traduit un niveau de ploïdie compatible avec une hybridation ultérieure et correspond aux ségrégeants ayant intégré le gène STL1.

La détermination de la présence de l'allèle MATalpha est réalisée par PCR. Cette réaction est réalisée en utilisant comme matrice l'ADN génomique de chaque ségrégeant potentiel. Les oligonucléotides sont d'une part une amorce propre au locus MAT exprimé sur le chromosome III de la levure (amorce Mat1) et deux autres amorces l'une propre à MATalpha (amorce Mat2) et l'autre propre à MATa (amorce Mat3). Le génotype MATalpha des cellules de levure se caractérise par la présence d'un amplicon de 404 paires de base détectables sur gel d'agarose à 0,8 %.

L'amorce Mat1 a la séquence SEQ ID N0: 2 suivante : AGTCACATCAAGATCGTTTATGG.

L'amorce Mat2 a la séquence SEQ ID N0: 3 suivante : GCACGGAATATGGGACTACTTCG.

L'amorce Mat3 a la séquence SEQ ID N0: 4 suivante : ACTCCACTTCAAGTAAGAGTTTG.

### Etape 6 : Sélection des ségrégeants de la souche de levure G

Les ségrégeants de la souche de levure G sont sélectionnés selon deux critères. Le premier est la présence de l'allèle MATa qui traduit un niveau de ploïdie compatible avec une hybridation ultérieure avec le ségrégeant de la souche de levure F. Le second critère est la force fermentative de ces ségrégeants.

La détermination de la présence de l'allèle MATa est réalisée par PCR. Cette réaction est réalisée en utilisant comme matrice l'ADN génomique de chaque ségrégeant potentiel. Les oligonucléotides sont d'une part une amorce propre au locus MAT exprimé sur le chromosome III de la levure (amorce Mat1 de séquence SEQ ID N0: 2) et deux autres amorces l'une propre à MATalpha (amorce Mat2 de séquence SEQ ID N0: 3) et l'autre propre à MATa (amorce Mat3 de séquence SEQ ID N0: 4).

Le génotype MATa des levures se caractérise par la présence d'un amplicon de 544 paires de base détectable sur gel d'agarose à 0,8 %.

La force fermentative des ségrégeants est déterminée comme décrit ci-dessus.

Les ségrégeants ayant une force fermentative supérieure à 100 ml sont sélectionnés.

### Etape 7 : Croisement et sélection des hybrides

Une öse d'un ségrégeant haploïde MATalpha est ensemencée en surface de la gélose d'une boîte de Pétri de milieu A. Une öse d'un ségrégeant haploïde MATa est mélangée au dépôt du ségrégeant MATalpha.

La composition du milieu A est la suivante : 10 g d'extrait de levure, 10 g de peptone, 20 g de glucose, 20g d'agar, pH 6,2 +/- 0,2, eau qsp IL.

La boîte de Pétri est ensuite mise en incubation pendant 24 heures à 30°C. Une öse du mélange est ensemencée en surface de la gélose d'une boîte de Pétri de milieu A. Cette étape est réitérée à 5 reprises. Lors du dernier ensemencement, les cellules sont striées de sorte à obtenir des colonies isolées, correspondant chacune à une souche de levure.

Sur chaque isolement, les cellules de levure sont cultivées de sorte à pouvoir en extraire l'ADN génomique puis une PCR est réalisée de sorte à vérifier que, dans une même cellule, les allèles MATa et MATalpha sont bien présents. Cette réaction est réalisée en utilisant comme matrice l'ADN génomique de chaque hybride potentiel.

Les oligonucléotides sont d'une part une amorce propre au locus MAT exprimé sur le chromosome III de la levure (amorce Mat1 de séquence SEQ ID N0: 2) et deux autres amorces l'une propre à Mat alpha (amorce Mat2 de séquence SEQ ID N0: 3) et l'autre propre à Mat a (amorce Mat3 de séquence SEQ ID N0: 4). Le génotype MATalpha/a des souches de levure se caractérise par la présence de deux amplicons de 404 et 544 paires de base détectables sur gel d'agarose à 0,8 %.

La capacité des souches de levure ainsi obtenues à se multiplier, en condition aérobie, dans un milieu de culture comprenant un mélange de glycérol et de sucre, pour obtenir des levures, puis la force fermentative des levures ainsi obtenues en pâte sans sucre sont alors évaluées.

### Résultats

L'objectif de cet exemple est d'obtenir une souche de levure améliorée capable de se multiplier en condition aérobie dans un milieu contenant du glycérol et du glucose, en consommant le glycérol, et donnant des levures possédant une force fermentative permettant leur utilisation en panification.

La souche de départ F est une souche de *Saccharomyces cerevisiae* génétiquement modifiée par intégration du gène STL1 dans une région liée au locus MAT de type sexuel MATalpha.

Cette simple modification génétique permet à la souche de départ F de se multiplier, en condition aérobie, dans un milieu de culture contenant du glycérol et du sucre, en consommant le glycérol.

Après sporulation, les ségrégeants du type sexuel MATalpha de la souche de levure F sont sélectionnés par PCR.

Afin d'améliorer les performances en panification de la souche de levure F, elle est croisée avec une souche de départ G qui est une souche de *Saccharomyces cerevisiae* non génétiquement modifiée et qui donne des levures performantes en panification sur des pâtes sans sucre.

Après sporulation de la souche de départ G, des ségrégeants de type sexuel MATa sont sélectionnés par PCR, puis une deuxième sélection est effectuée parmi ces ségrégeants du type sexuel MATa sur la base de leur force fermentative.

Les ségrégeants sélectionnés de la souche de levure F et de la souche de levure G sont alors hybridés.

Ainsi, le procédé utilisé est rapide, simple de mise en oeuvre et efficace :
- les ségrégeants MATalpha de la souche de levure F sont sélectionnés en une seule étape, sans avoir besoin de vérifier à la fois la présence du gène STL1 et le type sexuel du ségrégeant, et
- seules les forces fermentatives des ségrégeants du type sexuel MATa de la souche de levure G sont évalués, au lieu d'évaluer la force fermentative de tous les ségrégeants issus de la souche de levure G.

## Revendications

1. Procédé d'obtention et de sélection d'une souche de levure améliorée, par intégration d'au moins deux gènes d'intérêt puis croisement, comprenant les étapes de:
- sélection d'une souche de levure X hétérothallique et hétérozygote au niveau du locus d'expression du type sexuel,
- intégration dans le génome de ladite souche de levure X d'au moins deux gènes d'intérêt dans une région liée à un locus d'expression du type sexuel, pour obtenir une souche de levure X génétiquement modifiée, et
- croisement de la souche de levure X génétiquement modifiée avec une souche de levure Y, pour obtenir un hybride,
où l'intégration est effectuée avec une cassette d'intégration de gènes, ou un vecteur comprenant une cassette d'intégration de gènes, la cassette d'intégration de gènes comprenant:
- au moins deux cassettes d'expression, chaque cassette d'expression comprenant un gène d'intérêt encadré par un promoteur et un terminateur,
- deux séquences de recombinaison homologues d'une région liée à un locus d'expression du type sexuel et encadrant lesdites cassettes d'expression,
**caractérisée en ce que** ladite région liée à un locus d'expression du type sexuel est une région située à moins de 5 cM dudit locus d'expression du type sexuel et où l'étape de croisement de la souche de levure X génétiquement modifiée avec une souche de levure Y comprend les étapes de :
a) sporulation de la souche de levure X génétiquement modifiée et sélection, en une seule étape, d'au moins un ségrégeant du type sexuel correspondant à l'intégration des aux moins deux gènes d'intérêt, pour obtenir au moins un ségrégeant X,
b) sporulation de la souche de levure Y et sélection d'au moins un ségrégeant de la souche de levure Y de type sexuel compatible avec le ségrégeant X, pour obtenir un ségrégeant Y, et
c) hybridation d'au moins un ségrégeant X avec au moins un ségrégeant Y, pour obtenir au moins un hybride.

2. Procédé selon la revendication 1, **caractérisée en ce que** ladite région liée à un locus d'expression du type sexuel est une région située à moins de 10500 paires de base dudit locus d'expression du type sexuel.

3. Procédé selon la revendication 1 ou la revendication 2, **caractérisé en ce que** ladite cassette comprend en outre, entre lesdites deux séquences de recombinaison, deux séquences lox encadrant une cassette d'expression, ladite cassette d'expression comprenant un marqueur de sélection encadré par un promoteur et un terminateur.

4. Procédé selon l'une quelconque des revendications 1 à 3, **caractérisé en ce que** ladite région liée à un locus d'expression du type sexuel est comprise dans le gène BUD5, dans le gène TAF2 et/ou dans le locus d'expression du type sexuel.

5. Procédé selon l'une quelconque des revendications 1 à 4, **caractérisé en ce que** la souche de levure Y comprend au moins un gène d'intérêt dans une région liée à un locus d'expression du type sexuel.

6. Procédé selon l'une quelconque des revendications 1 à 5, **caractérisé en ce que** la souche de levure Y a au moins un caractère d'intérêt.

7. Procédé selon la revendication 6, **caractérisée en ce que** l'étape b) de croisement comprend en outre la sélection d'un ségrégeant de la souche de levure Y qui a tout ou partie dudit caractère d'intérêt, pour obtenir un ségrégeant Y.

8. Procédé selon la revendication 6 ou la revendication 7, **caractérisée en ce qu'**il comprend une étape ultérieure de sélection d'au moins un hybride qui a tout ou partie dudit caractère d'intérêt de la souche de levure Y.

## Patentansprüche

1. Verfahren zum Erhalt und zur Selektion eines verbesserten Hefestammes durch Integrieren von wenigstens zwei interessierenden Genen und anschließendem Kreuzen, umfassend die Schritte:
- Selektion eines heterothallischen und heterozygoten Hefestammes X bezüglich des Expressionslocus vom sexuellen Typ,
- Integration von wenigstens zwei interessierenden Genen in das Genom des Hefestammes X in einem mit einem Expressionslocus vom sexuellen Typ verbundenen Bereich, zum Erhalt eines genetisch veränderten Hefestamm X, und
- Kreuzen des gentechnisch veränderten Hefestammes X mit einem Hefestamm Y zum Erhalt eines Hybrids,
wobei die Integration mit einer Genintegrationskassette oder einem Vektor, der eine Genintegrationskassette umfasst, durchgeführt wird, wobei die Genintegrationskassette umfasst:
- wenigstens zwei Expressionskassetten, wobei jede Expressionskassette ein interessierendes Gen enthält, das von einem Promotor und einem Terminator eingerahmt ist,
- zwei homologe Rekombinationssequenzen eines mit einem Expressionslocus vom sexuellen Typ verbundenen Bereichs, die Expressionskassetten einrahmend,
**dadurch gekennzeichnet, dass** der mit einem Expressionslocus vom sexuellen Typ verbundene Bereich, ein Bereich ist, der weniger als 5 cM von dem Expressionslocus vom sexuellen Typ entfernt ist, und wobei der Schritt des Kreuzens des genetisch modifizierten Hefestammes X mit einem Hefestamm Y die Schritte umfasst:
a) Sporulation des gentechnisch veränderten Hefestammes X und Selektion wenigstens eines Segreganten vom sexuellen Typ, entsprechend der Integration der zwei interessierenden Gene, in einem einzigen Schritt zum Erhalt wenigstens eines Segreganten X,
b) Sporulation des Hefestammes Y und Selektion wenigstens eines Segreganten des Hefestammes Y vom sexuellen Typ, der mit dem Segreganten X kompatibel ist, zum Erhalt eines Segreganten Y, und
c) Hybridisierung wenigstens eines Segreganten X mit wenigstens einem Segreganten Y, zum Erhalt wenigstens eines Hybrids.

2. Verfahren nach Anspruch 1, **dadurch gekennzeichnet, dass** der mit einem Expressionslocus vom sexuellen Typ verbundene Bereich ein Bereich ist, der weniger als 10500 Basenpaare von dem Expressionslocus vom sexuellen Typ entfernt ist.

3. Verfahren nach Anspruch 1 oder Anspruch 2, **dadurch gekennzeichnet, dass** die Kassette ferner zwischen den beiden Rekombinationssequenzen zwei lox-Sequenzen umfasst, die eine Expressionskassette einrahmen, wobei die Expressionskassette einen von einem Promotor und einem Terminator umrahmten Selektionsmarker umfasst.

4. Verfahren nach einem der Ansprüche 1 bis 3, **dadurch gekennzeichnet, dass** der mit einem Expressionslocus vom sexuellen Typ verbundene Bereich, im BUD5-Gen, im TAF2-Gen und/oder im Expressionslocus vom sexuellen Typ enthalten ist.

5. Verfahren nach einem der Ansprüche 1 bis 4, **dadurch gekennzeichnet, dass** der Hefestamm Y wenigstens ein interessierendes Gen in einem mit einem Expressionslocus vom sexuellen Typ verbundenen Bereich umfasst.

6. Verfahren nach einem der Ansprüche 1 bis 5, **dadurch gekennzeichnet, dass** der Hefestamm Y wenigstens ein interessierendes Merkmal aufweist.

7. Verfahren nach Anspruch 6, **dadurch gekennzeichnet, dass** Schritt b) des Kreuzens zum Erhalt eines Segreganten Y ferner die Selektion eines Segreganten des Hefestammes Y umfasst, der das gesamte oder einen Teil des interessierenden Merkmals aufweist.

8. Verfahren nach Anspruch 6 oder Anspruch 7, **dadurch gekennzeichnet, dass** es einen nachfolgenden Schritt zur Selektion wenigstens eines Hybrides umfasst, der das gesamte oder einen Teil des interessierenden Merkmals des Hefestamms Y aufweist.

## Claims

1. Method for obtaining and selecting an improved strain of yeast by integrating at least two genes of interest and then by crossing, comprising the steps of:
- selecting a yeast strain X which is heterothallic and heterozygous at the level of the sex type expression locus,
- integrating, in the genome of said yeast strain X, at least two genes of interest into a region linked to a sex type expression locus, so as to obtain a genetically modified yeast strain X, and
- crossing the genetically modified yeast strain X with a yeast strain Y, so as to obtain a hybrid, where the integration is carried out with a gene integration cassette, or a vector comprising a gene integration cassette, the gene integration cassette comprising:
- at least two expression cassettes, each expression cassette comprising a gene of interest framed by a promoter and a terminator,
- two recombination sequences which are homologues of a region linked to a sex type expression locus and which frame said expression cassettes,
**characterized in that** said region linked to a sex type expression locus is a region located at least 5 cM from said sex type expression locus,
and where the step of crossing the genetically modified yeast strain X with a yeast strain Y comprises the steps of:
a) sporulation of the genetically modified yeast strain X and selection, in a single step, of at least one segregant of the sex type corresponding to the integration of the at least two genes of interest, so as to obtain at least one segregant X,
b) sporulation of the yeast strain Y and selection of at least one segregant of the yeast strain Y of sex type compatible with the segregant X, so as to obtain a segregant Y, and
c) hybridization of at least one segregant X with at least one segregant Y, so as to obtain at least one hybrid.

2. Method according to Claim 1, **characterized in that** said region linked to a sex type expression locus is a region located less than 10 500 base pairs from said sex type expression locus.

3. Method according to Claim 1 or Claim 2, **characterized in that** said cassette also comprises, between said two recombination sequences, two lox sequences framing an expression cassette, said expression cassette comprising a selectable marker framed by a promoter and a terminator.

4. Method according to any one of Claims 1 to 3, **characterized in that** said region linked to a sex type expression locus is included in the BUD5 gene, in the TAF2 gene and/or in the sex type expression locus.

5. Method according to any one of Claims 1 or 4, **characterized in that** the yeast strain Y comprises at least one gene of interest in a region linked to a sex type expression locus.

6. Method according to any one of Claims 1 to 5, **characterized in that** the yeast strain Y has at least one characteristic of interest.

7. Method according to Claim 6, **characterized in that** the crossing step b) also comprises selecting a segregant of the yeast strain Y which has all or part of said characteristic of interest, so as to obtain a segregant Y.

8. Method according to Claim 6 or Claim 7, **characterized in that** it comprises a subsequent step of selecting at least one hybrid which has all or part of said characteristic of interest of the yeast strain Y.


### LISTAGE DE SEQUENCES

<110> Lesaffre et Compagnie
<120> Procédé d'obtention de souches de levure améliorées
<130> DEM102FR
<160> 4
<170> PatentIn version 3.5
<210> 1
   <211> 34
   <212> DNA
   <213> Bacteriophage P1
<400> 1
   ataacttcgt ataatgtatg ctatacgaag ttat 34
<210> 2
   <211> 23
   <212> DNA
   <213> Saccharomyces cerevisiae
<400> 2
   agtcacatca agatcgttta tgg 23
<210> 3
   <211> 23
   <212> DNA
   <213> Saccharomyces cerevisiae
<400> 3
   gcacggaata tgggactact tcg 23
<210> 4
   <211> 23
   <212> DNA
   <213> Saccharomyces cerevisiae
<400> 4
   actccacttc aagtaagagt ttg 23